# EUROPEAN PATENT SPECIFICATION

(11) **EP 1 453 566 B1**
(45) Date of publication and mention of the grant of the patent: **03.09.2008**
(21) Application number: 02787624.2
(22) Date of filing: 08.11.2002
(51) Int. Cl.: A61M 25/00, A61M 27/00

(54) **AORTIC CANNULA**
AORTAKANÜLE
CANULE AORTIQUE

(30) Priority: 13.11.2001 IT MI20012395
(43) Date of publication of application: 08.09.2004
(73) Proprietor: Politecnico Di Milano, 20133 Milano (IT)
(72) Inventor: FUMERO, Roberto, 20080 Basiglio (IT); COSTANTINO, Maria Laura, 20090 Segrate (IT); FIORE, Gianfranco Beniamino, 20136 Milano (IT); GUADAGNI, Gualtiero, 20143 Milano (IT)
(74) Representative: Mittler, Enrico
(86) International application number: PCT/EP2002/012532
(87) International publication number: WO 2003/041782

(56) References cited:
- EP-A- 0 618 059
- US-A- 1 596 754
- US-A- 4 406 656
- US-A- 4 601 713
- US-A- 4 738 666
- US-A- 5 084 033

## Description

The present invention refers to a cannula, in particular an aortic cannula utilised in circuits for the extracorporeal treatment of blood or other fluid as for instance in the circuit for cardiopulmonary bypass during open heart surgery operations.

Aortic cannulae currently available on the market are essentially made up of ducts, whose end (tip) is inserted in the aorta through a surgically made slit. The tip of the cannulae is usually rigid and has a small cross section size as compared with the section of the aorta. The reason of this small size resides in the need to reduce the occlusion of the lumen of the aorta caused by the cannula. In fact when the heart starts beating again, a large sized obstacle in the aorta would cause a remarkable increase in the hydraulic resistance that the heart has to overcome. This could cause an excessive cardiac work because of the increase in the hydraulic load and it could possibly lead to heart failure.

On the other hand it is not possible to reduce the size of the cross section of the tip below a certain limit. In fact, when operating at full regime, the cannula is passed through by the entire blood flow that must be returned to the patient: by reducing its cross section it is possible to cause excessive pressure drop, serious mechanical damage to the red cells (haemolysis), damage of the aortic wall or undesired dislodging of atheromatous material from the wall of the vessel, effect caused by the kinetic energy of the fluid jet that is injected at a high speed (sand-blasting effect). Normally, a good compromise between these two requirements is thought to be obtained by choosing a cannula that occupies the cross section of the aorta by a portion not greater than 20-30%. For example, in the case of an adult patient, cannulae with tips having diameters of approximately 6-8 millimetres are used.

The aortic cannula is intended for use in extracorporeal treatments requiring blood to be processed at flowrates representing the patient's whole cardiac output, or a large fraction of it. The most frequent such application is cardio-pulmonary bypass (CPB), where the patient's heart pumping function is completely replaced by an external heart-lung machine, to allow surgical access to the non-beating heart. This involves, for example, exchanging blood at up to 61/min flowrates in an adult patient. Other applications are left and/or right ventricle circulatory support, extracorporeal membrane oxygenation (ECMO), extracorporeal carbon dioxide removal (ECCO2R), etc., where blood is processed at flowrates representing a large fraction of the patient's cardiac output.

In all these applications, the patient is surgically prepared to provide direct access to central arterial vessels. In CPB the aortic cannula is usually placed in the ascending aorta, immediately downstream of the aortic valve orifice.

The tip length is normally kept as short as possible in order to limit the mentioned hydraulic hindrance downstream of the heart. For example, for an adult patient, the tip length is normally in the range 1 to 5 cm.

Diametric and longitudinal dimensions are decreased, with respect to the adult dimensions, according to the patient size, e.g. in the cases of newborn or pediatric patients.

The tip can have different configurations. The most widely used cannulae are the ones with a straight beveled tip or with a curved tip.

A particular solution of tip is the one described in US patent 5,616,137 that describes a tip made of a flexible material having helical slits along its walls and a cap that partially or totally blocks the outflow hole. When the pressure of the fluid inside the cannula increases the slits get open and the fluid's outflow speed decreases.

US patent 5,868,717 discloses a partially collapsible dual lumen catheter related to fluid exchange therapies, such as hemodialysis or therapeutic apheresis. The catheter is intended for vascular access through relatively peripheral vessels. The dual lumen catheter has one lumen wall able to collapse against the other lumen in response to external, physiological body pressure when the catheter is in a latency phase, i.e., when it is not in use. Further cathoters with variable diameter are disclosed in US-A-4406656, US-A-473866 or US-A-1596754.

In view of the state of the art herein described, a scope of the present invention is to provide a tip with better performances as compared with the ones of the tips of the known art.

According to the present invention, these and other scopes are attained by means of an aortic cannula comprising a tubular tip, suitable to be inserted into an aorta, characterised in that said tubular tip comprises at least a first circumferential portion, made up of deformable material, and at least a second circumferential portion, made up of non deformable material, that extend longitudinally starting from the final end of said tubular tip, said second circumferential portion being placed adjacent to the aorta wall, on the same side of the surgically made slit, through which the tubular tip (1) is inserted in the aorta, so that, while a first fluid is input into said aorta through said cannula, said portion of deformable material stretches out so as to favour the flow of said first fluid and, while a second fluid is supplied by the heart, said portion of deformable material gets deformed thus reducing the section of said tubular tip so as to favour the flow of said second fluid.

Owing to the present invention it is possible to provide a cannula having a tip that can vary its geometry on the bases of the fluid mechanics requirements. In particular, such cannula guarantees to infuse high flow rates without causing high kinetic energy jets, since it makes available a passage section that is comparable to the one of the natural aorta, without even producing excessive resistance on behalf of the heart during the weaning transient (restart of the heart), owing to the deformability of the tip wall. These peculiarities allow overcoming one of the main technological limits that current state of the art devices suffer from, that is the fact of not being optimised neither for regime operating conditions, nor for the operating conditions during the transitory.

In particular, when it is the extracorporeal circuit that delivers all, or the preponderant part, of the blood flow rate, the tip is stretched out up to occupying a large part of the aortic section available (approximately 80%-90%), thus considerably reducing the disadvantages due to the reduced section. When instead it is also or only the heart that pumps blood to the patient the wall collapses up until occupying a negligible section of the aorta (approximately 5%-20%), thus reducing in this way the hydraulic resistance opposed to the cardiac pumping.

The characteristics and the advantages of the present invention will become evident from the following detailed description of an embodiment thereof, that is illustrated as a non-limiting example in the enclosed drawings, in which:
Figure 1A is a schematic layout of an embodiment of a tip, in one of the possible positions of installation, according to the present invention;
Figure 1B is a schematic layout of the front view in Figure 1A;
Figure 1C is a schematic perspective layout of Figure 1A;
Figure 2A is a schematic layout of an embodiment of the tip, in position of maximum expansion, according to the present invention;
Figure 2B is a schematic layout of the front view of Figure 2A;
Figure 2C is a schematic perspective layout of Figure 2A;
Figure 2D is a schematic layout in section taken along the plane of line IID in Figure 2B;
Figure 3A is a schematic layout of an embodiment of a tip, in position of minimum expansion, according to the present invention;
Figure 3B is a schematic layout of the front view of Figure 3A;
Figure 3C is a schematic perspective layout of Figure 3A;
Figure 4 is a schematic layout of a section of the final portion of the tip, in position of maximum expansion, according to another embodiment.

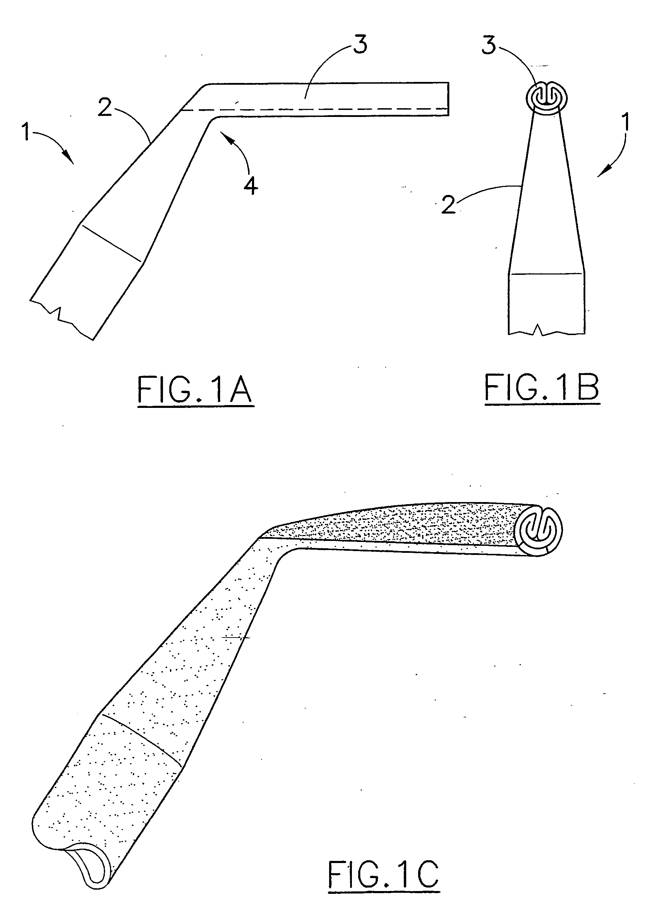

Figures 1A and 1B are schematic layouts of an embodiment of a tip of a cannula (not shown) according to the present invention. The tip 1 in this embodiment is of the type having a curve, at point 4, so as to facilitate its introduction in the aorta as also to withhold the tip in place during operation. The tip axes upstream and downstream of point 4 form an angle comprised between 90° (tip at right angle) and 180° (rectilinear tip), preferably between 120° and 150°.

In this embodiment, the tip 1 is made up of a body 2 made of a substantially rigid first material and a body 3 made of a substantially deformable second material.

The bodies 2 and 3 consist of distinct portions of the circumference of the final end of the tip 1 that extend longitudinally from the final end of the tip 1 for a pre-established length, typically up to the curvature point 4.

In this embodiment, the rigid body 2, in the zone downstream of point 4, as compared with the circumference of the tip 1 has a size comprised between approximately 0 and 50 % and preferably smaller than approximately 15%, and it is located on the internal side of the curvature of the tip, fixed to the completely rigid body 2 upstream of point 4. It has the functions to keep the cannula in its position during operation, to guarantee a correct positioning, and to allow its installation. The deformable body 3 takes the majority of the circumference of the tip, comprised between approximately 50 and 100% and preferably greater than approximately 85 %, and it gives it the property to modify the section according to the fluid dynamic requirements.

In Figures 1A, 1B and 1C the deformable body 3 is folded over onto itself thus occupying the smallest possible space so that it can be introduced in the aorta without major problems.

In the 2A figures, 2B, 2C and 2D the deformable body 3 is stretched and it has a maximum cross section.

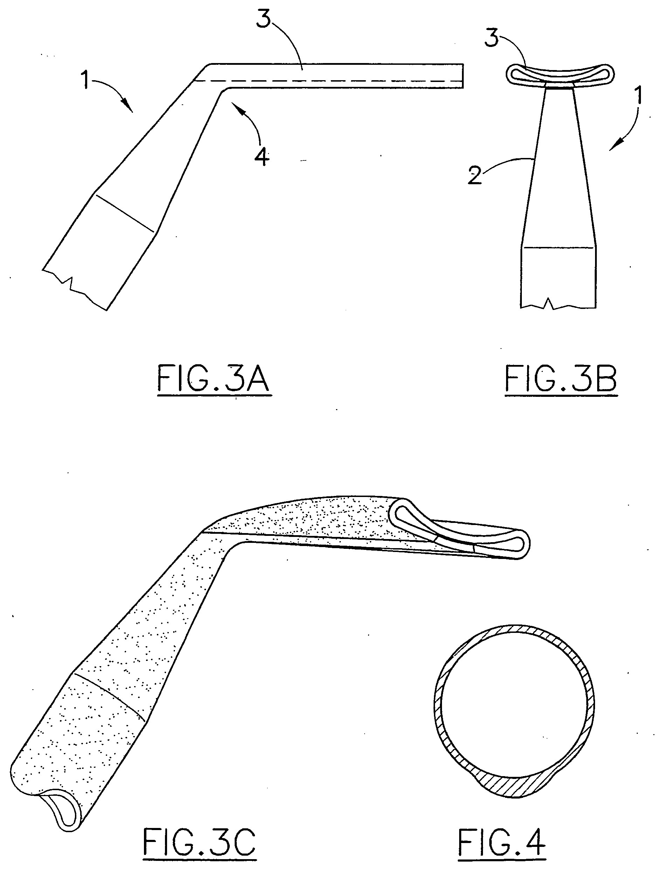

In Figures 3A, 3B and 3C the deformable body 3 is collapsed and it has a minimum cross section.

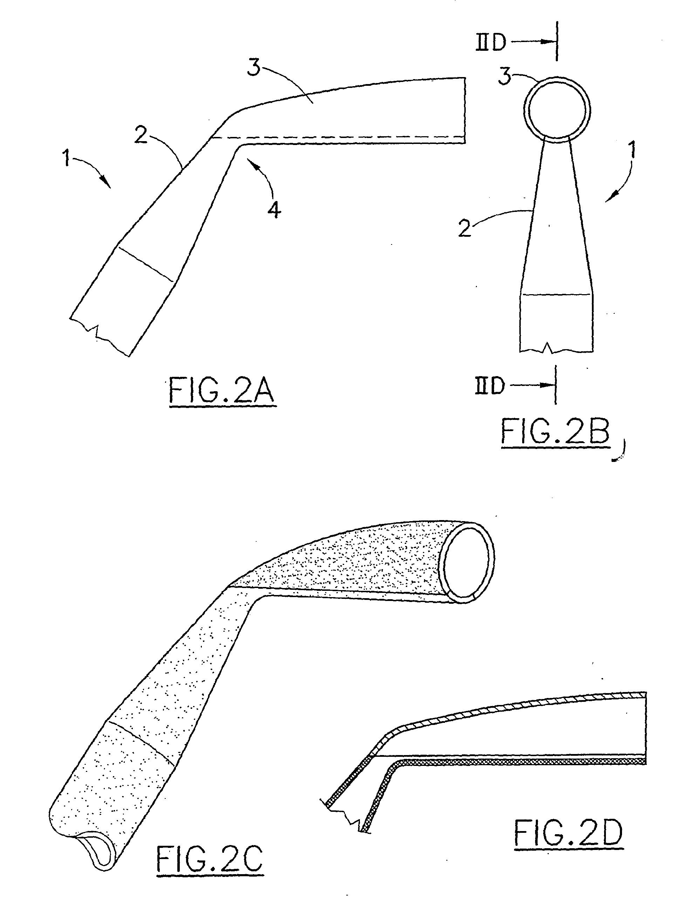

The tip 1, in the portion downstream of point 4, when the deformable body 3 is stretched as in Figures 2, is preferably made up of a substantially conical tubular duct, in such a way so as to increase the cross section available for the flow of the blood infused into the aorta, therefore to reduce the outflow speed avoiding the formation of a high energy jet, thus diminishing the shear stress within the fluid and the forces that the fluid transmits to the wall of the vessel.

The substantially conical portion of Figure 2, downstream of point 4, has an opening angle such that when the blood flow is delivered by the cannula 1, its final end occupies a considerable portion of the section of the aorta. In the preferred configuration, this portion is greater than approximately 80%, as opposed to the 20-30% portion typically occupied by the cannulae of the state of the art: in this way the average output speed of the fluid from the cannula is reduced by approximately 3-4 times and the kinetic energy associated to the flow by approximately 9-16 times.

In Figure 3 the deformable body 3 is shown as collapsed when the blood flow is delivered also by the heart, it occupies a negligible section of the aorta, approximately 5%-20%, thus reducing in this way the hydraulic resistance against cardiac pumping.

The opening or the collapse of the cannula tip are respectively determined by the fact that a fluid flow coming from the extracorporeal circuit pushes, by virtue of its kinetic energy, onto the inner surface of the deformable body or, vice versa, a fluid flow caused by the ventricular ejection pushes onto the outer surface of the deformable body. Such changes in shape of the deformable body are therefore due to a dynamic phenomenon caused by the movement of the fluid, rather than to static pressure differences only.

The tip is placed in the aorta in such way that the fluid flow coming from the extracorporeal circuit or from the natural heart only interact with the tip's deformable body. The rigid body does not interfere with such interaction, however it constitutes the means for the correct cannula placement.

Advantageously, the cannula placement in the vessel is unequivocally determined by the fact that the circumferential rigid portion, that is also rigid in the axial direction, is adjacent to the aorta wall, on the same side of the slit, made surgically, through which the tubular tip is inserted in the aorta, occupying a reduced or negligible percentage of the aortic lumen section. Consequently, the circumferential deformable portion, when operating and therefore in its expanded state, occupies a substantial percentage of the aorta section, extending itself inside said aorta section towards the side opposite to the slit entry of the tubular tip in the aorta, that is towards the axis of the aorta. In the preferred embodiment, the operator determines from the outside the correct positioning on the fact that the circumferential rigid portion constitutes the continuation of the external body of the cannula on the internal bending side of the tubular tip. In a possible tubular rectilinear tip configuration, the correct position can be determined by some proper marker placed on the external body of the cannula.

In the preferred embodiment, the tip 1 has a wall made up of two parts, a rigid one 2 and a deformable one 3. However a plurality of rigid and deformable modules can be provided along the perimeter of the section, as for instance several rigid modules interconnected to each other by deformable modules. Or a completely deformable tip can be provided, thus giving to an external element the function of keeping the cannula in position.

The tip 1, and in particular the deformable body 3 has preferably a substantially conical shape but it can also have a substantially cylindrical shape. The cross section of the tip 1 has preferably round shape but it can also have another shape as for example an elliptic shape.

The relative relationships between surface occupied by the rigid wall and by the deformable wall, the opening of the cone, the inclination angle of the tip and the relative radii of curvature are parameters that are subject to possible variations during the stage of optimisation of the device and may possibly vary with the size of the patient, in fact by its own nature, aortic cannulae are produced in different sizes as a function of the size of the patients which they are destined to.

The portion of the cannula external to the aorta can have any configuration among the ones that are normally used. In particular, it could either provide or not specific accessories suitable to the anchorage of the aorta, for the prevention of possible disadvantages such as kinking of the external tube, etc.

In the preferred embodiment, the tip 1 of the cannula is made of a resilient and flexible polymer material. The different rigidity of the parts of the tip are given by means of addition of additive to the polymer and by maintaining the relative thickness of the two parts unchanged, as in Figures 1, 2 and 3, or as an alternative, by means of variations in the thickness of the wall as it can be seen in Figure 4, that represents a schematic layout of a section of the final portion, downstream of point 4, of the tip 1 in a maximum expansion position.

Instead of the polymer material, it is possible to use a metal as for instance a very flexible one, in this case too the different rigidity would be given by means of variations of the thickness.

As an alternative it is possible to use a relatively rigid material (as for instance a metal) with a relatively flexible material (as for instance, a polymer), and binding the rigid part to the flexible one by means of gluing or welding, or to make the rigid part, onto which a layer of flexible material is superimposed, or also to make the flexible part with one or more stiffening inserts drowned into it.

The system of installation provides that at the time of packaging the deformable body 3 is collapsed or is refolded onto itself, as in Figure 1, so as to occupy the smallest possible space, and it is kept in such position by appropriate means that will be better specified later, up until the moment in which the surgeon, after having introduced the cannula in the aorta activates it.

One packaging method is the one by which the cannula lumen is put in depression and its proximal section gets closed by a watertight plug, in such a way that the entire deformable wall of the tip stays completely collapsed, as in Figures 1, up to the moment in which the surgeon, after having introduced the cannula in the aorta, increases the pressure inside the deformable body 3 by removing the aforesaid plug.

As an alternative, it is possible to use a semi-rigid containing sheath that gets removed by the surgeon after its installation. Or to use one or more suture stitches as a system for holding the deformable body 3, bound by means of slipknots, either prepared during the packaging stage or by the surgeon during surgery. Or yet maintaining the deformable body 3 collapsed through gluing by means of soluble and highly bio-compatible glues (starches and sugars), that dissolve in the blood shortly after the installation. Or to maintain the deformable body 3 collapsed owing to characteristic property of the material that it is made of, as for instance, using a shape memory material a rigid cannula could be made that is collapsed at room temperature and that at operating temperature becomes deformable and patent again. Or yet to use active systems that are controlled from the outside.

In the preferred embodiment, the behaviour of the deformable tip is completely passive, that is the deformation is guided exclusively by the fluid flow pushing on its inner or outer surfaces.

The possibility to adopt active systems is not excluded. Active systems due to characteristics of the materials themselves, as for instance: the use of activable materials, that is that they change their mechanical properties according to an external stimulus (for example: polymer conductors activated by means of application of electric potential), the use of materials whose mechanical characteristics vary considerably when a critical value of temperature is exceeded (for example: shape memory polymers or alloys). Systems activated from the outside, as for instance activation by mechanics: bars, rods, etc., magnetic or electromagnetic activation.

The cannula herein described especially for aortic use, can also be used for others applications as for instance for ventricular assistance with or without counter-pulsation, other circulatory support treatments such as ECMO or ECCO2R.

The present invention therefore provides a cannula with variable geometry that as far as the geometry of the duct external to the aorta is concerned, is equivalent to a conventional cannula. Its peculiarity instead consists in having a tip whose wall is deformable for a considerable portion of its circumference so that its shape varies spontaneously the size of its section is considerably reduced when fluid flow pushes onto the outer tip surfaces. In this way, the geometry of the duct that makes up the tip is suitable to all possible fluid mechanics conditions of operation.

## Claims

1. Aortic cannula comprising a tubular tip (1), suitable to be inserted into an aorta, **characterised in that** said tubular tip (1) comprises at least a first circumferential portion, made up of deformable material (3), and at least a second circumferential portion, made up of non deformable material (2), that extend longitudinally starting from the final end of said tubular tip (1), said second circumferential portion (2) comprises a positioning means in order to place it adjacent to the aorta wall, on the same side of the surgically made slit, through which the tubular tip (1) is inserted in the aorta, so that, while a first fluid is introduced intro said aorta through said cannula, said portion of deformable material (3) stretches out so as to favour the flow of said first fluid and, while a second fluid is supplied by the heart, said portion of deformable material (3) deforms thus reducing the section of said tubular tip (1) so as to favour the flow of said second fluid.

2. Aortic cannula according to claim 1 **characterised in that** said deformable circumferential portion (3) has a size comprised between 50 and 100% as compared with the circumference of said tubular tip (1), and preferably greater than 85%.

3. Aortic cannula according to claim 1 **characterised in that** it comprises at least one circumferential portion that extends longitudinally and made up of non deformable material (2) that has a size comprised between 0 and 50% as compared with the circumference of said tubular tip (1), and preferably smaller than 15%.

4. Aortic cannula according to claim 1 **characterised in that** said tubular tip (1) has substantially conical shape with the larger section at the final end of said tubular tip (1).

5. Aortic cannula according to claim 1 **characterised in that** said tubular tip (1) has substantially cylindrical shape.

6. Aortic cannula according to claim 1 **characterised in that** said deformable material (3) and said non deformable material (2) are made of the same material with different thickness.

7. Aortic cannula according to claim 1 **characterised in that** said deformable material (3) and said non deformable material (2) are made of material having substantially the same thickness but different rigidity.

8. Aortic cannula according to claim 1 **characterised in that** said deformable material (3) and said non deformable material (2) are made up of different materials having different rigidity.

9. Aortic cannula according to claim 1 **characterised in that** said tip (1) in position of installation before being inserted in the aorta has said deformable material (3) refolded onto itself, so as to occupy the smallest possible space.

10. Aortic cannula according to claim 9 **characterised in that** said deformable material (3) refolded onto itself is kept in such position by means of a containing sheath.

11. Aortic cannula according to claim 9 **characterised in that** said deformable material (3) refolded onto itself is kept in such position by means of a soluble and bio-compatible glue.

12. Aortic cannula according to claim 9 **characterised in that** said deformable material (3) refolded onto itself is kept in such position by means of depression.

13. Aortic cannula according to claim 9 **characterised in that** said deformable material (3) refolded onto itself is kept in such position by means of suture stitches held by slipknots.

14. Aortic cannula according to claim 1 **characterised in that** said tubular tip (1) is of the type having a bend, said second circumferential portion (2) being placed on the internal bending side of said tubular tip (1).

## Patentansprüche

1. Aortakanüle, welche eine röhrenförmige Spitze (1), die zum Einführen in eine Aorta geeignet ist, aufweist,
**dadurch gekennzeichnet, dass**
die röhrenförmige Spitze (1) zumindest einen ersten umlaufenden Abschnitt bestehend aus verformbarem Material (3) und zumindest einen zweiten umlaufenden Abschnitt bestehend aus nicht-verformbarem Material (2), die Abschnitte sich vom Ende der röhrenförmige Spitze (1) beginnend längs erstrecken, aufweist, und der zweite umlaufende Abschnitt (2) ein Positioniermittel aufweist, um ihn an der Aortawand auf derselben Seite des chirurgisch geschaffenen Schlitzes, durch welchen die röhrenförmige Spitze (1) in die Aorta eingesetzt ist, zu platzieren, so dass, während ein erstes Fluid in die Aorta durch die Kanüle eingeführt wird, sich der Abschnitt aus verformbaren Material (3) ausdehnt, um den Fluss des ersten Fluids zu begünstigen und, während ein zweites Fluid durch das Herz zugeführt wird, sich der Abschnitt aus verformbaren Material (3) verformt und auf diese Weise den Querschnitt der röhrenförmigen Spitze (1) verringert, um den Fluss des zweiten Fluids zu begünstigen.

2. Aortakanüle nach Anspruch 1, **dadurch gekennzeichnet, dass** der verformbare, umlaufende Abschnitt (3) eine Größe hat, die in einem Bereich zwischen 50 und 100%, und vorzugsweise mehr als 85%, des Umfangs der röhrenförmigen Spitze (1) liegt.

3. Aortakanüle nach Anspruch 1, **dadurch gekennzeichnet, dass** sie zumindest einen umlaufenden Abschnitt aufweist, welcher sich längs erstreckt und aus einem nicht-verformbaren Material (2) besteht und eine Größe hat, die in einem Bereich zwischen 0 und 50%, und vorzugsweise weniger als 15%, des Umfangs der röhrenförmigen Spitze (1) liegt.

4. Aortakanüle nach Anspruch 1, **dadurch gekennzeichnet, dass** die röhrenförmige Spitze (1) im Wesentlichen kegelförmig mit dem größeren Querschnitt am Ende der röhrenförmigen Spitze (1) ist.

5. Aortakanüle nach Anspruch 1, **dadurch gekennzeichnet, dass** die röhrenförmige Spitze (1) im Wesentlichen zylinderförmig ist.

6. Aoartakanüle nach Anspuch 1 **dadurch gekennzeichnet, dass** das verformbare Material (3) und das nicht-verformbare Material (2) aus demselben Material mit unterschiedlicher Dicke bestehen.

7. Aortakanüle nach Anspruch 1 **dadurch gekennzeichnet, dass** das verformbare Material (3) und das nicht-verformbare Material (2) aus einem Material mit einer im Wesentlichen gleichen Dicke aber unterschiedlicher Steifigkeit bestehen.

8. Aortakanüle nach Anspruch 1, **dadurch gekennzeichnet, dass** das verformbare Material (3) und das nicht-verformbare Material (2) aus unterschiedlichen Materialien mit unterschiedlicher Steifigkeit bestehen.

9. Aortakanüle nach Anspruch 1, **dadurch gekennzeichnet, dass** vor dem Einsetzen in die Aorta das verformbare Material (3) der Spitze (1) in ihrer Einsetzposition in sich selbst gefaltet ist, um den kleinstmöglichen Platz einzunehmen.

10. Aortakanüle nach Anspruch 9, **dadurch gekennzeichnet, dass** das in sich selbst gefaltete, verformbare Material (3) mittels einer Haltehülle in einer solchen Position gehalten ist.

11. Aortakanüle nach Anspruch 9, **dadurch gekennzeichnet, dass** das in sich selbst gefaltete, verformbare Material (3) mittels eines löslichen und biokompatiblen Klebers in einer solchen Position gehalten ist.

12. Aortakanüle nach Anspruch 9, **dadurch gekennzeichnet, dass** das in sich selbst gefaltete, verformbare Material (3) mittels Unterdruck in einer solchen Position gehalten ist.

13. Aortakanüle nach Anspruch 9, **dadurch gekennzeichnet, dass** das in sich selbst gefaltete, verformbare Material (3), mittels chirurgischen Nähten, die durch Laufknoten gehalten sind, in einer solchen Position gehalten ist.

14. Aortakanüle nach Anspruch 1, **dadurch gekennzeichnet, dass** die röhrenförmige Spitze (1) mit einer Krümmung ausgeführt ist, wobei der zweite umlaufende Abschnitt (2) auf der innen liegenden Krümmungsseite der röhrenförmigen Spitze (1) angeordnet ist.

## Revendications

1. Canule aortique comprenant une pointe tubulaire (1), adaptée pour être insérée dans une aorte, **caractérisée en ce que** ladite pointe tubulaire (1) comprend au moins une première portion circonférentielle, faite de matériau déformable (3) et au moins une seconde portion circonférentielle, faite de matériau non déformable (2), qui s'étend longitudinalement en partant de l'extrémité finale de ladite pointe tubulaire (1), ladite seconde portion circonférentielle (2) comprenant un système de positionnement permettant de la placer de manière adjacente à la paroi de l'aorte, du même côté que la fente réalisée chirurgicalement, à travers laquelle la pointe tubulaire (1) est insérée dans l'aorte, de sorte que, pendant qu'un premier fluide est introduit dans ladite aorte à travers ladite canule, ladite portion de matériau déformable (3) s'étire vers l'extérieur, de façon à favoriser le flux dudit premier fluide et, tandis qu'un second fluide est fourni par le coeur, ladite portion de matériau déformable (3) se déforme, en réduisant ainsi la section de ladite pointe tubulaire (1), de façon à favoriser le flux dudit seconde fluide.

2. Canule aortique selon la revendication 1, **caractérisée en ce que** ladite portion circonférentielle déformable (3) a une taille comprise entre 50 et 100 %, par rapport à la circonférence de ladite pointe tubulaire (1), et de préférence supérieure à 85 %.

3. Canule aortique selon la revendication 1, **caractérisée en ce qu'**elle comprend au moins une portion circonférentielle qui s'étend longitudinalement et faite de matériau non déformable (2), qui a une taille comprise entre 0 et 50 % par rapport à la circonférence de ladite pointe tubulaire (1), et de préférence inférieure à 15 %.

4. Canule aortique selon la revendication 1, **caractérisée en ce que** ladite pointe tubulaire (1) a une forme sensiblement conique avec la section la plus grande à l'extrémité finale de ladite pointe tubulaire (1).

5. Canule aortique selon la revendication 1, **caractérisée en ce que** ladite pointe tubulaire (1) a une forme sensiblement cylindrique.

6. Canule aortique selon la revendication 1, **caractérisée en ce que** ledit matériau déformable (3) et ledit matériau non déformable (2) sont faits du même matériau mais avec une épaisseur différente.

7. Canule aortique selon la revendication 1, **caractérisée en ce que** ledit matériau déformable (3) et ledit matériau non déformable (2) sont faits de matériau ayant sensiblement la même épaisseur mais une rigidité différente.

8. Canule aortique selon la revendication 1, **caractérisée en ce que** ledit matériau déformable (3) et ledit matériau non déformable (2) sont faits de différents matériaux ayant une rigidité différente.

9. Canule aortique selon la revendication 1, **caractérisée en ce que** ladite pointe (1) en position d'installation, avant d'être insérée dans l'aorte, présente ledit matériau déformable (3) replié sur lui-même, de façon à occuper le moins d'espace possible.

10. Canule aortique selon la revendication 9, **caractérisée en ce que** ledit matériau déformable (3) replié sur lui-même est maintenu dans une telle position au moyen d'une gaine de contention.

11. Canule aortique selon la revendication 9, **caractérisée en ce que** ledit matériau déformable (3) replié sur lui-même est maintenu dans une telle position au moyen d'une colle soluble et biocompatible.

12. Canule aortique selon la revendication 9, **caractérisée en ce que** ledit matériau déformable (3) replié sur lui-même est maintenu dans une telle position au moyen d'une dépression.

13. Canule aortique selon la revendication 9, **caractérisée en ce que** ledit matériau déformable (3), replié sur lui-même est maintenu dans une telle position au moyen de points de suture maintenus par des noeuds coulissants.

14. Canule aortique selon la revendication 1, **caractérisée en ce que** ladite pointe tubulaire (1) est de type ayant une pliure, ladite seconde portion circonférentielle (2) étant placée du côté du pliage interne de ladite pointe tubulaire (1).
